# EUROPEAN PATENT APPLICATION

(11) **EP 0 743 520 A2**
(43) Date of publication of application: **20.11.1996**
(21) Application number: 96111192.9
(22) Date of filing: 29.12.1994
(51) Int. Cl.: G01N 33/50, C12Q 1/68

(54) **Novel uses for GAL4-receptor constructs**

(30) Priority: 30.12.1993 US 177740
(62) Divisional of application: 95905946.0
(71) Applicant: THE SALK INSTITUTE FOR BIOLOGICAL STUDIES, La Jolla California 92037 (US)
(72) Inventor: Evans, Ronald M., La Jolla, CA 92037 (US); Umesono, Kazuhiko, La Jolla, CA 92037 (US); Blumberg, Bruce, San Diego, CA 92122 (US); Rangarajan, Pundi N., Bangalore 560079 (IN)
(74) Representative: Kolb, Helga, Dr. Dipl.-Chem.

(57) **Abstract**

In accordance with the present invention, it has been discovered that chimeric receptors comprising at least the ligand binding domain of a member of the steroid/thyroid receptor protein superfamily having incorporated therein the DNA binding domain of the yeast GAL4 protein are capable of forming functional homodimers. Such homodimers are useful, for example, in assays to determine the ligand for an orphan member of the steroid/thyroid superfamily of receptors, for identifying compounds which are selective for a specific steroid/thyroid receptor protein from among a plurality of such receptors which respond to a common ligand, and the like.

## Description

### ACKNOWLEDGEMENT

This invention was made with Government support under Grant Nos. HD27183, awarded by the National Institute of Health. The Government has certain rights in the invention.

### FIELD OF THE INVENTION

The present invention relates to intracellular receptors, and ligands therefor. In a particular aspect, the present invention relates to methods for the identification of compounds which function as ligands (or ligand precursors) for intracellular receptors. In another aspect, the present invention relates to methods for the identification of compounds which are selective for a specific receptor protein from among a plurality of receptors which respond to a common ligand. In a further aspect, the present invention relates to novel chimeric intracellular receptors and uses therefor.

### BACKGROUND OF THE INVENTION

A central problem in eukaryotic molecular biology continues to be the elucidation of molecules and mechanisms that mediate specific gene regulation. As part of the scientific attack on this problem, a great deal of work has been done in efforts to identify ligands (i.e., exogenous inducers) which are capable of mediating specific gene regulation.

Although much remains to be learned about the specifics of gene regulation, it is known that ligands modulate gene transcription by acting in concert with intracellular components, including intracellular receptors and discrete DNA sequences known as hormone response elements (HREs).

As additional members of the steroid/thyroid superfamily of receptors are identified, the search for exogenous inducers for such newly discovered receptors (i.e., naturally occurring (or synthetic) inducers) has become an important part of the effort to learn about the specifics of gene regulation.

The identification of compounds which directly or indirectly interact with intracellular receptors, and thereby affect transcription of hormone-responsive genes, would be of significant value, e.g., for therapeutic applications.

Additional novel intracellular receptors (i.e., members of the steroid/thyroid superfamily of receptors) continue to be identified. Frequently, however, the primary ligand(s) for these novel receptors can not readily be identified. Accordingly, methods for the ready identification of ligands for such receptors would be of great value.

Of the intracellular receptors for which ligands have been identified, there are some which respond to a common ligand (e.g., retinoic acid receptor (RAR) and retinoid X receptor (RXR) both respond to the presence of retinoic acid; glucocorticoid receptor (GR) and mineralocorticoid receptor (MR) both respond to the presence of glucocorticoids, etc.). In order to modulate process(es) mediated by one, but not all of the receptor subtypes which respond to a common ligand, a great deal of energy has been invested in efforts to discover ligands which are selective for a single subtype. It is difficult, however, to differentiate between the actions of a single ligand on several different receptor subtypes. Accordingly, methods to identify compounds selective for a single subtype would also be of great value.

It has recently been discovered that some intracellular receptors are functional for regulation of transcription only when associated with another member of the steroid/thyroid superfamily of receptors (i.e., as a heterodimer). Until the physiological processes impacted by a specific receptor are known, however, it is not possible to determine if an orphan receptor functions as a homodimer or requires a partner (so as to form a functional heterodimer). Accordingly, means to simplify the characterization of orphan receptors (i.e., receptors for which no ligand has yet been identified), without the need to determine if said orphan requires a partner to produce a functional receptor, would also be of great value.

Other information helpful in the understanding and practice of the present invention can be found in commonly assigned United States Patent Nos. 5,071,773 and 4,981,784; and United States Patent Application Nos. 325,240, filed March 17, 1989; 370,407, filed June 22, 1989; and 438,757, filed November 16, 1989, all of which are hereby incorporated herein by reference in their entirety.

### BRIEF DESCRIPTION OF THE INVENTION

In accordance with the present invention, we have discovered that chimeric receptors comprising a member of the steroid/thyroid receptor protein superfamily having incorporated therein the DNA binding domain of the yeast GAL4 protein are capable of forming functional homodimers. Such homodimers are useful, for example, in assays to determine the ligand for an orphan member of the steroid/thyroid superfamily of receptors, for identifying compounds which are selective for a specific steroid/thyroid receptor protein from among a plurality of such receptors which respond to a common ligand, and the like.

Such assays have a broad dynamic range and very low background signals, since the response system triggered by the presence of suitable ligand is not endogenous to the cells employed for the assays. A response is obtained only when the chimeric receptors of the invention are activated, without competition from endogenous receptors which may be present in the test cells.

In accordance with another embodiment of the present invention, methods are provided for the identification of compounds which are convertible into ligand or ligand precursors for intracellular receptors, or other compounds which may induce receptors through a non-ligand activated pathway. In accordance with yet another embodiment of the present invention, methods are provided for the identification of compounds (e.g., enzymes) which are capable of converting appropriate precursor compounds into ligands for intracellular receptors (or other compounds which may induce receptors through a non-ligand activated pathway). In accordance with still another embodiment of the present invention, methods are provided for the identification of cell lines (or active fractions thereof) which are capable of promoting the above-described conversions.

### BRIEF DESCRIPTION OF THE FIGURES

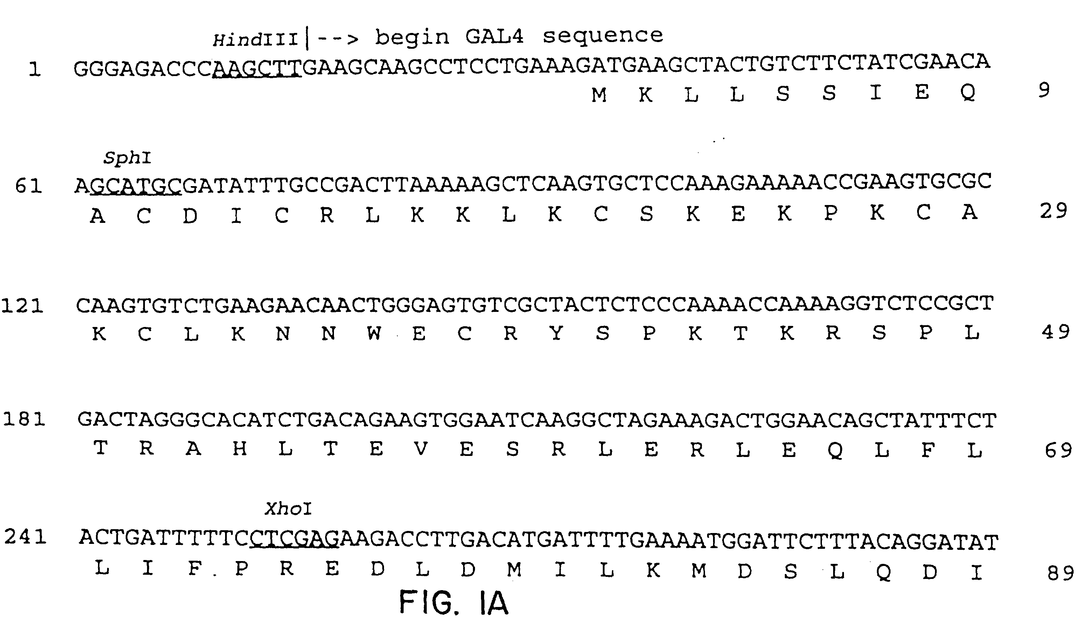

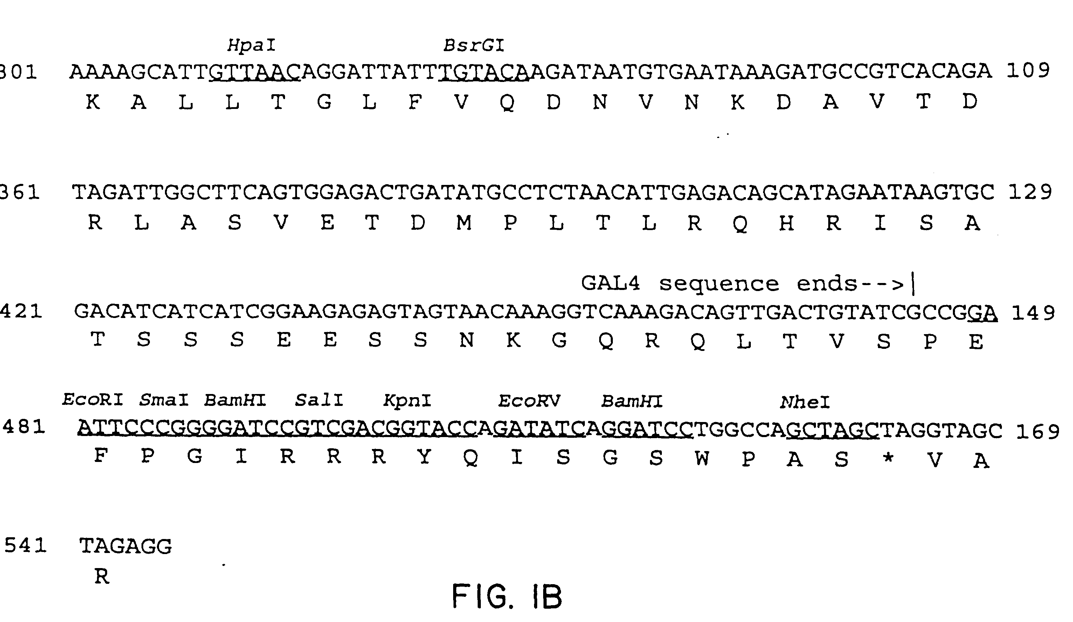

Figure 1 is a restriction map of the GAL4 encoding portion of vector pCMX-GAL4 employed in the preparation of GAL4-receptor fusion proteins.

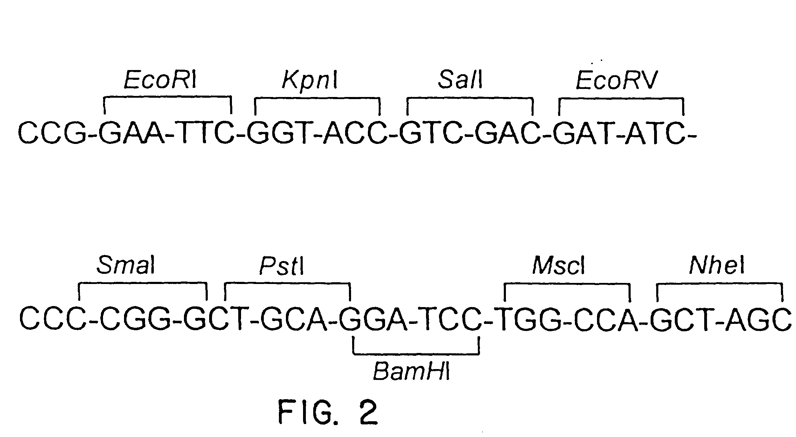

Figure 2 is a restriction map of the linker set forth in SEQ ID NO:4.

Figure 3 is a dose response curve for various hRARα constructs, in the presence of all trans retinoic acid. In the figure, ◇ designates unmodified hRARα, ◆ designates the chimeric receptor GRR (i.e., a chimeric receptor wherein the amino terminal domain is derived from the glucocorticoid receptor and the DNA and ligand binding domains are derived from RAR), □ designates the chimeric receptor GGR (i.e., a chimeric receptor wherein the amino terminal domain and the DNA binding domain are derived from GR, and the ligand binding domain is derived from RAR), and ■ designates GAL4-hRARα.

### DETAILED DESCRIPTION OF THE INVENTION

In accordance with the present invention, there is provided a method to determine the ligand or ligand precursor for an orphan member of the steroid/thyroid receptor protein superfamily, said method comprising
contacting cells containing a modified form of said orphan member with a putative ligand for said orphan member;
   wherein said modified form of said orphan member is produced by introducing the DNA binding domain of GAL4 into said orphan member, and
   wherein said cells contain a GAL4 response element operatively linked to a reporter gene, and thereafter
monitoring the expression of reporter gene product.
Optionally, the modified receptor employed in the practice of the present invention will also contain one or more exogenous transactivation domains, such as, for example, the τ₁ or τ₂ transactivation domains described in United States Patent No. 5,217,867, which is incorporated by reference herein in its entirety.

Invention assay method is especially useful where the response element for said orphan receptor has not been identified and/or where the ability of said receptor to function in vivo as a homodimer or a heterodimer is unknown.

Thus, in accordance with another embodiment of the present invention, there is provided a method to render a steroid/thyroid receptor protein capable of forming a homodimer, said method comprising introducing the DNA binding domain of GAL4 into said receptor protein.

As employed herein, the term "ligand or ligand precursor for a member of the steroid/thyroid receptor protein superfamily" (i.e., intracellular receptor) refers to a substance or compound which (in its unmodified form, or after conversion to its "active" form), inside a cell, binds to the receptor protein, thereby creating a ligand/receptor complex, which in turn can activate an appropriate hormone response element. A ligand therefore is a compound which acts to modulate gene transcription for a gene maintained under the control of a hormone response element, and includes compounds such as hormones, growth substances, non-hormone compounds that modulate growth, and the like. Ligands include steroid or steroid-like hormone, retinoids, thyroid hormones, pharmaceutically active compounds, and the like. Individual ligands may have the ability to bind to multiple receptors.

Accordingly, as employed herein, "putative ligand" refers to compounds such as steroid or steroid-like hormones, pharmaceutically active compounds, and the like, which are suspected to have the ability to bind to the orphan receptor of interest, and to modulate transcription of genes maintained under the control of response elements recognized by such orphan receptor.

Examples of known ligands include all-*trans*retinoic acid (ligand for retinoic acid receptor), 9-*cis*-retinoic acid (ligand for retinoid X receptor), dexamethasone (ligand for glucocorticoid receptor), thyroid hormone (ligand for thyroid hormone receptor), 1,25-dihydroxy vitamin D₃ (ligand for vitamin D₃ receptor), and the like.

Examples of known ligand precursors include all-*trans*-retinoic acid (which can be converted to 9-*cis*-retinoic acid) , 25-hydroxy vitamin D₃ (which can be converted to 1,25-dihydroxy vitamin D₃), β-carotene, biologically active lipids (e.g., fatty acids such as linoleic acid or arachidonic acid), and the like.

As employed herein, the term "members of the steroid/thyroid receptor protein superfamily" (also known as "nuclear receptors" or "intracellular receptors") refers to hormone binding proteins that operate as ligand-dependent transcription factors. Members of the steroid/thyroid receptor protein superfamily for which specific ligands have not yet been identified are referred to herein as "orphan receptors". These hormone binding proteins have the intrinsic ability to bind to specific DNA sequences. Following binding, the ligand/orphan receptor complex functions to modulate the transcriptional activity of target gene (i.e., a gene associated with the specific DNA sequence).

It is useful to distinguish the terms receptor "subtype" and receptor "class". For example, retinoid responsive receptors comprise a "class" of receptors, all of which are responsive to retinoid compounds. Similarly, thyroid hormone receptors comprise a "class" of receptors which are responsive to thyroid hormone. Each class can be divided into various subtypes, i.e., specific members of the class which have different tissue distributions, different affinities for the native ligand, different activation properties when contacted with the native ligand, and so on.

Some classes of receptors include sub-families of distinctly different types of receptors. Thus, for example, while the retinoid class of receptors includes both the retinoic acid receptors (RARs) and the retinoid X receptors (RXRs), these two different sub-families are clearly distinct. For example, each member of the RAR sub-family is responsive to a defined first hormone response element (HRE), and each member of the RXR sub-family is responsive to a defined second HRE (which is distinctly different from the first HRE). Accordingly, in accordance with one aspect of the present invention, there is provided a method to identify compounds which are selective for a specific steroid/thyroid receptor protein from among a plurality of such receptors which respond to a common ligand (i.e., different subtypes of the same sub-family or members of the same class, but a different sub-family), said method comprising
introducing the DNA binding domain of GAL4 into each one of said plurality of receptor proteins, thereby producing a plurality of modified receptor proteins,
contacting cells containing one of said modified receptor proteins with a putative selective ligand for said modified receptor protein;
   wherein said cells contain a GAL4 response element operatively linked to a reporter gene,
monitoring the expression of reporter gene product, then
repeating said contacting and monitoring steps with a different modified receptor protein until all members of said plurality of modified receptor proteins have individually undergone said contacting, and thereafter
identifying those compounds which are selective for one specific steroid/thyroid receptor protein from among said plurality of receptors which respond to a common ligand.

There are a number of receptors which respond to a common ligand, thus the plurality f receptors contemplated by the above-described method can be selected from the multiple subtypes of a receptor responsive to a common ligand, e.g., the various subtypes of RAR and/or the various subtypes of RXR, the various subtypes of PPAR, the various subtypes of COUP, and the like.

Such compounds are useful, for example, for the treatment of steroid or steroid-like hormone-responsive disease states. As used herein, the phrase "steroid or steroid-like hormone responsive disease state" refers to:
(i) any disease state wherein a gene product (or a portion of a gene product) not normally subject to steroid or steroid-like hormone expression control is placed, by translocation, under the control of a steroid or steroid-like hormone responsive sequence, or
(ii) any disease state wherein a first gene product (or a portion of a gene product) subject to steroid or steroid-like hormone expression control by a first steroid or steroid-like hormone is placed, by translocation, under the control of a second steroid or steroid-like hormone responsive sequence, or
(iii) any disease state which correlates with the expression of abnormal gene product, wherein said gene product (or a portion of said gene product) is normally subject to steroid or steroid-like hormone expression control, or
(iv) any disease state which correlates with an abnormal level of expression of gene product, the expression of which is normally maintained under steroid or steroid-like hormone expression control, or
(v) any disease state which correlates with an abnormal level of receptor, the presence of which is normally maintained under steroid or steroid-like hormone expression control, or
(vi) any disease state which correlates with an abnormal level of ligand, the presence of which is normally maintained under steroid or steroid-like hormone expression control.

As employed herein, the phrase "compounds which are selective for a specific steroid/thyroid receptor protein from among a plurality of such receptors which respond to a common ligand" refers to compounds which interact with the receptor subtype associated with said steroid or steroid-like hormone responsive disease state to a significantly greater extent than with other subtypes or sub-families of the same receptor class, i.e., compounds which are preferentially selective for one receptor subtype (or sub-family) in modulating the transcription activation properties thereof. The terminology "significantly greater extent", as applied to interaction between ligand and a specific receptor subtype (or sub-family), refers to ligands which have a significantly higher therapeutic index (i.e., the ratio of efficacy to toxicity) for treatment of the target disease state than for activation of pathways mediated by other subtypes (or sub-families) of the same receptor class. The toxicity of therapeutic compounds frequently arises from the non-selective interaction of the therapeutic compound with receptor subtypes (or sub-families) other than the desired receptor subtype (or sub-family). Thus, the present invention provides a means to dramatically reduce the incidence of side-reactions commonly associated with hormone therapy.

The DNA-binding domains of all members of the steroid/thyroid receptor protein superfamily are related, consisting of 66-68 amino acid residues, and possessing about 20 invariant amino acid residues, including nine cysteines.

A member of the superfamily can be identified as a protein which contains the above-mentioned invariant amino acid residues, which are part of the DNA-binding domain of such known steroid receptors as the human glucocorticoid receptor (amino acids 421-486), the estrogen receptor (amino acids 185-250), the mineralocorticoid receptor (amino acids 603-668), the human retinoic acid receptor α1 (amino acids 88-153). The highly conserved amino acids of the DNA-binding domain of members of the superfamily are as follows: 
Cys - X - X - Cys - X - X - Asp* - X -
Ala* - X - Gly* - X - Tyr* - X - X -
X - X - Cys - X - X - Cys - Lys* -
X - Phe - Phe - X - Arg* - X - X - X -
X - X - X - X - X - X - (X - X -) Cys -
X - X - X - X - X - (X - X - X -) Cys -
X - X - X - Lys - X - X - Arg - X - X -
Cys - X - X - Cys - Arg* - X - X -
Lys* - Cys - X - X - X - Gly* - [?]et
(SEQ ID No 1);
 wherein X designates non-conserved amino acids within the DNA-binding domain; the amino acid residues denoted with an asterisk are residues that are almost universally conserved, but for which variations have been found in some identified hormone receptors; and the residues enclosed in parenthesis are optional residues (thus, the DNA-binding domain is a minimum of 66 amino acids in length, but can contain several additional residues).

Exemplary members of the steroid/thyroid superfamily of receptors include steroid receptors such as glucocorticoid receptor, mineralocorticoid receptor, progesterone receptor, androgen receptor, vitamin D₃ receptor, and the like; plus retinoid receptors, such as RARα, RARβ, RARγ, and the like, plus RXRα, RXRβ, RXRγ, and the like; thyroid receptors, such as TRα, TRβ, and the like; as well as other gene products which, by their structure and properties, are considered to be members of the superfamily, as defined hereinabove. Examples of orphan receptors include HNF4 [see, for example, Sladek et al., in Genes & Development 4: 2353-2365 (1990)], the COUP family of receptors [see, for example, Miyajima et al., in Nucleic Acids Research 16: 11057-11074 (1988), Wang et al., in Nature 340: 163-166 (1989), including the COUP-like receptors and COUP homologs, such as those described by Mlodzik et al., in Cell 60: 211-224 (1990) and Ladias et al., in Science 251: 561-565 (1991)], the ultraspiracle receptor [see, for example, Oro et al., in Nature 347: 298-301 (1990)], PPAR [see, for example, Dreyer et al., in Cell 68:879-887 (1992)], and the like.

As set forth herein, the DNA binding domain of GAL4 protein is utilized in the present invention in the identification of ligands or ligand-precursors for orphan receptors; and in the identification of compounds selective for a specific steroid/thyroid receptor protein from among a plurality of steroid/thyroid receptor proteins. The DNA binding domain of the yeast GAL4 protein comprises at least the first 74 amino acids thereof (see, for example, Keegan et al., Science 231:699-704 (1986)). Preferably, the first 90 or more amino acids of the GAL4 protein will be used, with the first 147 amino acid residues of yeast GAL4 being presently most preferred.

The GAL4 fragment employed in the practice of the present invention can be incorporated into any of a number of sites within a steroid/thyroid receptor protein. For example, the GAL4 DNA binding domain can be introduced at the amino terminus of a steroid/thyroid receptor protein, or the GAL4 DNA binding domain can be subs tuted for the native DNA binding domain of a steroid/thyroid receptor, or the GAL4 DNA binding domain can be introduced at the carboxy terminus of a steroid/thyroid receptor protein, or at other positions as can readily be determined by those of skill in the art. Thus, for example, a modified receptor protein can be prepared which consists essentially of amino acid residues 1-147 of GAL4, plus the ligand binding domain of said steroid/thyroid receptor protein (i.e., containing the ligand binding domain only of said receptor, substantially absent the DNA binding domain and amino terminal domain thereof).

Identification methods according to the present invention involve the use of a functional bioassay system, wherein the modified receptor and a reporter plasmid are cultured in suitable host cells in the presence of test compound. Evidence of transcription (e.g., expression) of reporter gene is then monitored to determine the presence of an activated receptor-ligand complex. Accordingly, the functional bioassay system utilizes two plasmids: an "expression" plasmid and a "reporter" plasmid. The expression plasmid can be any plasmid which contains and is capable of expressing DNA encoding the desired modified form of orphan steroid/thyroid receptor protein, in a suitable host cell. The reporter plasmid can be any plasmid which contains an operative GAL4 response element functionally linked to an operative reporter gene.

Exemplary GAL4 response elements are those containing the palindromic 17-mer: 5'-CGGAGGACTGTCCTCCG-3' (SEQ ID NO:5), such as, for example, 17MX, as described by Webster et al., in Cell 52:169-178 (1988), as well as derivatives thereof. Additional examples of suitable response elements include those described by Hollenberg and Evans in Cell 55:899-906 (1988); or Webster et al. in Cell 54:199-207 (1988).

Exemplary reporter genes include chloramphenicol transferase (CAT), luciferase (LUC), beta-galactosidase (β-gal), and the like. Exemplary promoters include the simian virus (SV) promoter or modified form thereof (e.g., ΔSV), the thymidine kinase (TK) promoter, the mammary tumor virus (MTV) promoter or modified form thereof (e.g., ΔMTV), and the like [see, for example, Mangelsdorf et al., in Nature 345:224-229 (1990), Mangelsdorf et al., in Cell 66:555-561 (1991), and Berger et al., in J. Steroid Biochem. Molec. Biol. 41:733-738 (1992)]. The plasmids pGMCAT, pGHCAT, pTK-GALₚ3-LUC, ΔMTV-GALₚ3-LUC, ΔMTV-GALₚ3-CAT, and the like, are examples of reporter plasmids which contain an operative hormone responsive promoter/enhancer element functionally linked to an operative reporter gene, and can therefore be used in the above-described functional bioassay (see Example 2 for details on the preparation of these plasmids). In pGMCAT, the operative hormone responsive promoter/enhancer element is the MTV LTR; in pGHCAT it is the functional portion of the growth hormone promoter. In both pGMCAT and GHCAT the operative reporter gene is the bacterial gene for chloramphenicol acetyltransferase (CAT).

As used herein in the phrase "operative response element functionally linked to an operative reporter gene", the word "operative" means that the respective DNA sequences (represented by the terms "GAL4 response element" and "reporter gene") are operational, i.e., work for their intended purposes; the word "functionally" means that after the two segments are linked, upon appropriate activation by a ligand-receptor complex, the reporter gene will be expressed as the result of the fact that the "GAL4 response element" was "turned on" or otherwise activated.

In practicing the above-described functional bioassay, the expression plasmid and the reporter plasmid are co-transfected into suitable host cells. The transfected host cells are then cultured in the presence and absence of a test compound to determine if the test compound is able to produce activation of the promoter operatively linked to the GAL4 response element of the reporter plasmid. Thereafter, the transfected and cultured host cells are monitored for induction (i.e., the presence) of the product of the reporter gene sequence.

Any cell line can be used as a suitable "host" for the functional bioassay contemplated for use in the practice of the present invention. Thus, in contrast to the requirements of prior art assay systems, there is no need to use receptor-negative cells in carrying out the invention process. Since the modified receptor employed in the practice of the present invention is the only species in the test cell which is capable of initiating transcription from a GAL4 response element, the expression of native receptor by the test cell does not contribute to background levels. Thus, the invention bioassay is very selective.

Cells contemplated for use in the practice of the present invention include transformed cells, non-transformed cells, neoplastic cells, primary cultures of different cell types, and the like. Exemplary cells which can be employed in the practice of the present invention include Schneider cells, CV-1 cells, HuTu80 cells, F9 cells, NTERA2 cells, NB4 cells, HL-60 cells, 293 cells, Hela cells, yeast cells, and the like. Preferred host cells for use in the functional bioassay system are COS cells and CV-1 cells. COS-1 (referred to as COS) cells are monkey kidney cells that express SV40 T antigen (Tag); while CV-1 cells do not express SV40 Tag. The presence of Tag in the COS-1 derivative lines allows the introduced expression plasmid to replicate and provides a relative increase in the amount of receptor produced during the assay period. CV-1 cells are presently preferred because they are particularly convenient for gene transfer studies and provide a sensitive and well-described host cell system.

The above-described cells (or fractions thereof) are maintained under physiological conditions when contacted with physiologically active compound. "Physiological conditions" are readily understood by those of skill in the art to comprise an isotonic, aqueous nutrient medium at a temperature of about 37°C.

In accordance with another embodiment of the present invention, there is provided a method of screening for antagonists of steroid/thyroid receptor proteins, said method comprising
culturing test cells containing
   (i) increasing concentrations of at least one compound whose ability to inhibit the transcription activation activity of hormone receptor agonists is sought to be determined, and
   (ii) optionally, at least one agonist for said steroid/thyroid receptor protein(s) or functional modified forms thereof,
wherein said test cells contain
   (i) exogenous DNA which expresses a modified form of a steroid/thyroid receptor protein(s) or functional modified forms thereof, wherein the modified form of the steroid/thyroid receptor protein(s) or functional modified forms thereof contains the DNA binding domain of GAL4, and
   (ii) a GAL4 response element operatively linked to a reporter gene; and thereafter
assaying for evidence of transcription of said reporter gene in said cells as a function of the concentration of said compound in said culture medium, thereby indicating the ability of said compound to inhibit activation of transcription by hormone receptor agonists.

Media employed for such culturing may include agonist for the receptor being tested, or the receptor may be constitutive (i.e., not require the presence of agonist for activation), or a fixed concentration of agonist can be added to the media employed for such testing.

The above-described assays of the present invention have low background and a broad dynamic range.

In accordance with yet another embodiment of the present invention, there is provided a method for identifying biologically active compounds characterized by being:
convertible into ligand or ligand precursor for intracellular receptor(s), or
capable of effecting transformation of a second compound into ligand or ligand precursor for intracellular receptor(s), or
capable of effecting transformation of an intracellular receptor into its active form,
said method comprising:

(1) contacting cells or cell fractions with said biologically active compound under physiological conditions;
(2) fractionating the cells, cell fractions and/or the medium in which said contacting is carried out; and
(3) subjecting each of said fractions to a functional assay wherein each fraction is cultured with host cells containing DNA encoding the above-described GAL4-receptor chimeric constructs, and a GAL4 response element operatively linked to reporter-encoded DNA, and expression of reporter gene is monitored to determine the presence of an activated receptor-ligand complex.

Examples of ligands and ligand precursors for intracellular receptor(s) have been set forth above. Compounds capable of effecting transformation of an intracellular receptor into its active form include compounds which act as activators of receptors by alternative biochemical pathways not involving direct interaction of the compound with the receptor. Examples of such compounds include dopamine, fatty acids, clofibric acid, xenobiotics, and the like.

A wide variety of cells are contemplated or use in the practice of this embodiment of the present invention, as noted above with respect to alternate embodiments of the invention.

Cell fractions contemplated for use in this embodiment of the present invention include a variety of subcellular fractions, e.g., nuclear fractions, cytosolic fractions, membrane fractions, and the like.

Fractionating the cells, cell fractions and/or medium in which the above-described contacting is carried out can be conducted in a variety of ways, as can be readily determined by those of skill in the art. For example, the fermentation broth can be extracted into aqueous and/or organic media and further fractionated and purified by a variety of physical separation techniques, such as, for example, standard column chromatography, high performance liquid chromatography (HPLC) separation, ultracentrifugation (e.g., density gradient or isopycnic banding), thin layer chromatography (TLC), and the like.

The invention identification method involves the use of a functional bioassay system, wherein each of the above-described fraction(s) is cultured with host cells containing DNA encoding the above-described GAL4-receptor chimeric constructs, and a GAL4 response element operatively linked to reporter-encoded DNA, and expression of reporter gene product is monitored to determine the presence of an activated receptor-ligand complex. Accordingly, the functional bioassay system utilizes two plasmids: an "expression" plasmid and a "reporter" plasmid. The expression plasmid can be any plasmid which contains and is capable of expressing the above-described GAL4-receptor chimeric constructs (or mutants thereof), in a suitable host cell. The reporter plasmid can be any plasmid which contains an operative GAL4 responsive promoter/enhancer element functionally linked to an operative reporter gene.

Exemplary reporter genes suitable for use in the practice of this embodiment of the present invention are as described above with respect to alternate embodiments of the invention.

When using the functional bioassay system to determine the functionality of ligands for intracellular receptors, i.e., members of the steroid/thyroid superfamily of receptors, in preferred forms, plasmids will carry a selectable marker such as the amp gene. In addition, in preferred forms the reporter plasmids will have a suitable hormone responsive promoter/enhancer element e.g., the MTV LTR or a functional portion of the growth hormone promoter.

Expression plasmids containing the SV40 origin of replication (ori) can propagate to high copy number in any host cell which expresses SV40 Tag. Thus expression plasmids carrying the SV40 "ori" can replicate in COS cells, but not in CV-1 cells. Although the increased expression afforded by high copy number is desired, it is not critical to the operability of the "cis-trans" bioassay system. The expression vectors typically employed in the bioassay are generally so efficient that the assay works in virtually any host.

Thus, as described above with respect to other embodiments of the present invention, any cell line can be used as a suitable "host" for the functional bioassay contemplated for use in this embodiment of the present invention.

Based on the above-described functional bioassays, the activation of receptor by steroids or steroid-like compounds (or the inhibition of such activation by test compounds in the presence of agonists) can be examined.

In accordance with still another embodiment of the present invention, there is provided a method for identifying cell lines which are capable of promoting the conversion of biologically active compounds into:
(A) ligand or ligand precursor for intracellular receptor(s), or
(B) species which promote the transformation of a second compound into ligand or ligand precursor for intracellular receptor(s), or
(C) species which are capable of effecting transformation of an intracellular receptor into its active form,
said method comprising:
(1) contacting cells with biologically active compound(s) under conditions suitable to maintain the viability of said cells;
(2) fractionating the cells and the medium in which the cells are maintained;
(3) subjecting each of said fractions to a functional bioassay wherein each fraction is cultured with host cells containing DNA encoding the above-described GAL4-receptor chimeric constructs, and a GAL4 response element operatively linked to reporter-encoded DNA, and expression of reporter gene is monitored to determine the presence of an activated receptor-ligand complex; and
(4) identifying those cell lines which promote the conversion of said biologically active compounds into products functional in said functional bioassay.

In accordance with a still further embodiment of the present invention, there is provided a method for identifying enzymatic or non-enzymatic activities capable of promoting the conversion of biologically active compounds into:
(A) ligand or ligand precursor for intracellular receptor(s), or
(B) species which promote the transformation of a second compound into ligand or ligand precursor for intracellular receptor(s), or
(C) species which are capable of effecting transformation of an intracellular receptor into its active form,
said method comprising:
(1) subjecting biologically active compound(s) to conditions suitable to promote the desired conversion;
(2) subjecting the reaction product(s) of step (1) to a functional bioassay wherein each fraction is cultured with host cells containing DNA encoding the above-described GAL4-receptor chimeric constructs, and a GAL4 response element operatively linked to reporter-encoded DNA, and expression of reporter gene is monitored to determine the presence of an activated receptor-ligand complex; and
(3) identifying those conditions which promote the conversion of said biologically active compounds into products functional in said functional assay as providing the desired activities.

Conditions suitable to promote the desired conversion of biologically active compound include contacting with cell fractions which potentially contain the desired activity, subjecting the biologically active compound to temperature variations, pH changes, ultraviolet radiation, or any other chemical or physical exposure which has the potential to effect a modification of the activity of the biologically active compound.

The invention will now be described in greater detail by reference to the following non-limiting examples.

### Example 1

### Preparation of GAL4-receptor fusion proteins

A basic vector useful for the generation of GAL4-receptor fusion proteins is called pCMX-GAL4 (see SEQ ID NO:2). This vector encodes GAL4 DNA binding domain, followed by a polylinker sequence useful in the cloning. The parental expression vector pCMX has been described by Umesono et al., in Cell 65:1255-1266 (1991), and the GAL4 portion of pCMX-GAL4 is derived from plasmid pSG424, described by Sadowski and Ptashne, in Nucleic Acids Res. 17:7539 (1989).

In general, GAL4-receptor ligand binding domain fusions are prepared by taking advantage of mutant receptor cDNA clones, such as GR-RAR chimera [see, for example, Giguere et al., in Nature 330:624-629 (1987)]. These mutant receptor cDNAs encode common *Xho*I sites at the end of the DNA binding domain, as described by Giguere et al., supra. To do so, a new pCMX-GAL4 vector was prepared which encodes a compatible *Sal*I site in the polylinker sequence (there is an *Xho*I site in the GAL4 sequence): 
SalI site:  G'TCGAC
XhoI site:  C'TCGAG
 This allows efficient transfer of the receptor ligand binding domain to GAL4 DNA binding domain. Through this method, a number of chimeric species have been generated, e.g., GAL4-hRARα, GAL4-hTRβ, GAL4-hGR, GAL4-hERR1, GAL4-hERR2, GAL4-hXR2 (hXR2 is described in co-pending application Serial No. 07/761,068, now pending), GAL4-mXR5 (mXR5 is described in co-pending application Serial No. 07/761,068, now pending), GAL4-hVDR, GAL4-hRXRα, and the like. For example, hRARα cDNA was mutated to encode a unique *Xho*I site as follows (see Giguere et al., supra): AAA-GAG (K155-E156) → CTC-GAG (L155-E156) This mutated RARα cDNA was then digested with *Xho*I and *Bam*HI, and ligated into a *Sal*I-*Bam*HI digest of pCMX-GAL4 (containing the "new" linker set forth in SEQ ID NO:4, wherein the linker set forth in SEQ ID NO:4 is inserted into the construct set forth in SEQ ID NO:2 in place of residues 479-532 thereof), to produce a construct having the sequence, in relevant part: GAL4 DBD---CCG-GAA-TTC-GGT-ACC-GTC-GAG-TCT-GTG---RARα LBD, wherein "DBD" refers to DNA binding domain, and "LBD" refers to ligand binding domain.

If mutants of the type referred to above are not available for the construction of GAL4-containing chimerics, one may simply look for any convenient restriction enzyme site downstream of the DNA binding domain of the receptor of interest (i.e., within about the first 30 amino acid residues downstream of the conserved Gly-Met residues of the DNA binding domain, i.e., within 30 residues of the last two residues shown in SEQ ID NO:1), and utilize the carboxy terminal sequences therefrom.

Alternatively, one can utilize PCR-based mutagenesis to add any desired restriction sites to a segment of DNA. For example, primers can be designed which are complementary to the ligand binding domain of any member of the steroid/thyroid superfamily of receptors. Such primers are capable of amplifying desired portions of a given receptor utilizing PCR. By adding the desired restriction sites to the ends of the primers, one can ensure that each copy synthesized by PCR will possess such restriction enzyme sites at its termini [Scharf, Cloning with PCR. *In: PCR Protocols* (Innis et al., eds.) Academic Press Inc. (San Diego, CA) 84-91 (1990)]. Subsequently, the fragment can be digested with both enzymes and ligated into pCMX-GAL4, which has been digested with the same or compatible enzymes.

Thus, for example, the ligand binding domain of chicken RARβ can be amplified using PCR with primers complementary to amino acids 147-154 and 447-452. A SalI site can be added to the 5' end and a NheI site to the 3' end. After amplification, the fragment can be digested with SalI and NheI, then ligated to pCMX-GAL4 (which has been digested with SalI and NheI; employing pCMX-GAL4n which contains the polylinker set forth in SEQ ID NO:4).

### Example 2

### Preparation of reporter constructs

Various reporter constructs are used in the examples which follow. They are prepared as follows:

TK-LUC: The MTV-LTR promoter sequence was removed from the MTV-LUC plasmid described by Hollenberg and Evans in Cell 55:899-906 (1988) by *Hind*III and *Xho*I digest, and cloned with the *Hind*III-*Xho*I fragment of the Herpes simplex virus thymidine kinase gene promoter (-105 to +51 with respect to the transcription start site, m, isolated from plasmid pBLCAT2, described by Luckow & Schutz in Nucleic Acids Res. 15:5490 (1987)) to generate parental construct TK-LUC.

pTK-TREp2-LUC: Two copies of double-stranded palindromic thyroid hormone response element (TREp) oligonucleotides, encoding a retinoic acid receptor (RAR) binding site, were cloned upstream of the TK promoter of TK-LUC at the *Hind*III site.

pTK-GALp3-LUC: Three copies of double-stranded 17MX oligonucleotides, encoding a GAL4 binding site, were cloned upstream of the TK promoter of TK-LUC at the *Hind*III site.

ΔMTV-GALp3-CAT: Three copies of double-stranded 17MX oligonucleotides, encoding a GAL4 binding site, were cloned at the *Hind*III site in the ΔMTV-CAT plasmid described by Hollenberg and Evans, supra.

ΔMTV-GALp3-LUC: Three copies of double-stranded 17MX oligonucleotides, encoding a GAL4 binding site, were cloned at the *Hind*III site in the ΔMTV-LUC plasmid described by Hollenberg and Evans, supra. The ΔMTV-LUC plasmid encodes a mutant MTV-LTR promoter which no longer responds to hormones, as described by Hollenberg and Evans, supra.

CMX-βGAL: The coding sequence for the *E. coli* β-galactosidase gene was isolated from plasmid pCH110 [see Hall et al., J. Mol. Appl. Genet. 2:101-109 (1983)] by *Hind*III and *Bam*HI digest, and cloned into pCMX eucaryotic expression vector [see Umesono et al., supra].

### Example 3

### Screening assay for receptor selective agonists

CV-1 cells are co-transfected with CMX-GAL-hRARα and tk-galₚx3-luc in 96 well plates. The usual amounts of DNA per 96 wells are 1 µg of CMX-GAL-hRARα, 5 µg of tk-galₚx3-luc, and 4 µg of CMX-βGAL. Typically, transfections are performed in triplicate. The plates are then incubated overnight at 37°C.

The cells are washed twice with fresh medium. Fresh medium containing one concentration of a serial dilution of agonist is added to each well. A typical agonist dilution series extends from 10⁻⁵M through 10⁻¹¹M. A solvent control is performed for each agonist. The cells are incubated at 37°C for 1-2 days.

The cells are rinsed twice with buffered saline solution. Subsequently, cells are lysed, *in situ,* by adding 200 µl of lysis buffer. After 30 minutes incubation at room temperature, 40 µl aliquots of cell lysate are transferred to 96-well plates for luciferase reporter gene assays and β-galactosidase transfection controls [see Heyman et al., Cell 68:397-406 (1992)].

The data are expressed as relative light units (RLUs) per O.D. unit of β-galactosidase per minute. The triplicates are averaged for each concentration and plotted as normalized RLUs against the dose of agonist or as fold induction vs the dose of agonist.

The selectivity of an agonist for a particular receptor is measured by comparing the activation of that receptor with the activation seen by the same agonist utilizing other related receptors. It is frequently useful to express the activation elicited by a particular agonist as a percent of the activation elicited by the maximum concentration of all-trans retinoic acid on the same receptor.

This example demonstrates the ease with which the process of the present invention can be adapted for screening large numbers of compounds and/or cell lines to determine the presence of ligand or the ability of a test cell line to produce a functional ligand from the components of the media.

### Example 4

### Dose response of various hRARα constructs

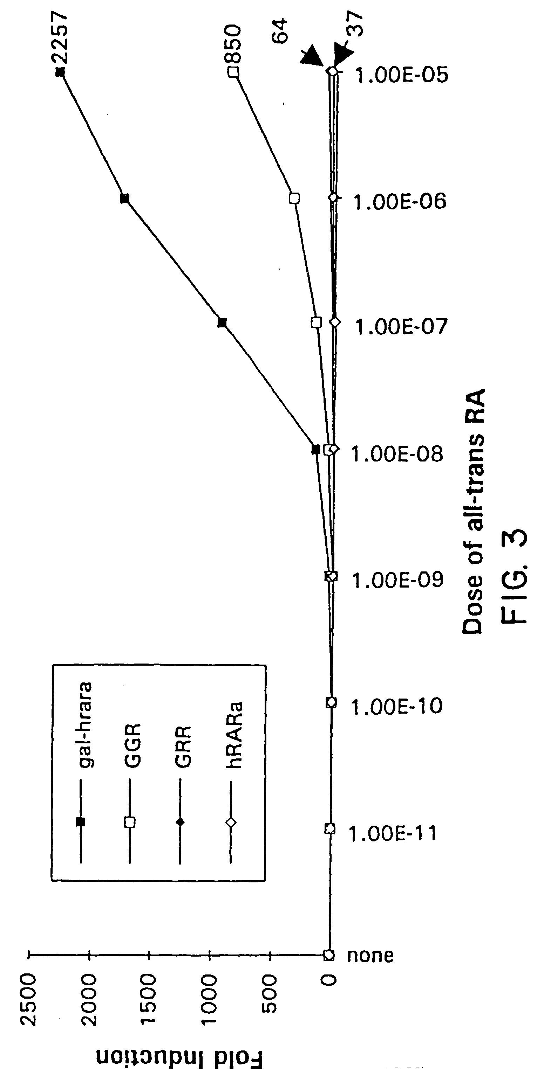

Effector plasmid, reporter plasmid, and β-galactosidase control plasmid are co-transfected into CV-1 cells at a ratio of about 1:5.4, using a liposome-mediated method, employing DOTAP (Boehringer Manheim) according to manufacturer's instructions in serum-free media (e.g., Opti-MEM, from Life Technologies, Gaithersberg, MD). After about 18 hours, the cells are washed twice with fresh Opti-MEM and all-trans retinoic acid is added to the media to the final molar concentration indicated in Figure 3. After 24 hours of incubation, the cells are rinsed with phosphate buffered saline (pH 7.2) and lysed. Aliquots are assayed for luciferase and β-galactosidase activity. Luciferase activity is normalized to optical density units of β-galactosidase per minute of incubation.

The data are expressed in Figure 3 as fold induction over the same construct incubated in solvent alone. The receptor:reporter combinations employed are as follows:
CMX-GAL-hRARα1 (wherein the ligand binding domain of hRARα is fused to amino acids 1-173 of GAL4) with pTK-GALp3-LUC;
RS-GGR (wherein the amino-terminal and DNA binding domains of human glucocorticoid receptor alpha are fused to the ligand binding domain of hRARα) with MTV-LUC;
RS-GRR (wherein the amino-terminal domain of human glucocorticoid receptor alpha is fused to the DNA binding and ligand binding domains of hRARα) with pTK-TREp2-LUC; and
CMX-hRARα1 with pTK-TREp2-LUC.

Review of Figure 3 reveals that chimeric receptors of the invention are much more responsive in agonist-type assays than are unmodified receptors, or chimeric receptors employed in the prior art (e.g., GRR or GGR).

While the invention has been described in detail with reference to certain preferred embodiments thereof, it will be understood that modifications and variations are within the spirit and scope of that which is described and claimed.

### Summary of Sequences

Sequence ID No. 1 is the consensus amino acid sequence of the DNA binding domain of members of the steroid/thyroid superfamily of receptors.

Sequence ID No. 2 is a nucleic acid sequence (and the deduced amino acid sequence) of a vector useful for the preparation of GAL4-receptor fusion proteins, including an exemplary segment encoding a GAL4 DNA binding domain useful in the practice of the present invention.

Sequence ID No. 3 is the deduced amino acid sequence of the nucleic acid sequence of SEQ ID NO:2.

Sequence ID No. 4 is the nucleic acid sequence of a linker useful in the preparation of chimeric constructs of the invention.

Sequence ID No. 5 is the nucleic acid sequence of a GAL4 response element, useful in the practice of the present invention.

## Claims

1. A method to determine the ligand or ligand precursor for an orphan member of the steroid/thyroid receptor protein superfamily, said method comprising
contacting cells containing a modified form of said orphan member with a putative ligand for said orphan member;
wherein said modified form of said orphan member is produced by replacing the DNA binding domain of said orphan member with the DNA binding domain of GAL4, and
wherein said cells contain a GAL4 response element operatively linked to a reporter gene, and thereafter
monitoring the expression of reporter gene product.

2. A method according to claim 1 wherein the DNA binding domain of GAL4 comprises amino acid residues 1-147 of GAL4.

3. A method according to claim 1 wherein the DNA binding domain of GAL4 is positioned at the amino terminus of the orphan member.

4. A method to identify compounds which are selective for a specific steroid/thyroid receptor protein from among a plurality of such receptors which respond to a common ligand, said method comprising
replacing the DNA binding domain of each one of said plurality of receptor proteins with the DNA binding domain of GAL4, thereby producing a plurality of modified receptor proteins,
individually contacting cells containing one of said modified receptor proteins with a putative selective ligand for said modified receptor protein until all members of said plurality of modified receptor proteins have individually undergone said contacting;
wherein said cells contain a GAL4 response element operatively linked to a reporter gene,
monitoring the expression of reporter gene product, and thereafter
identifying those compounds which are selective for one specific steroid/thyroid receptor protein from among said plurality of receptors which respond to a common ligand.

5. A method according to claim 4 wherein said plurality of receptor proteins comprises multiple subtypes of a receptor responsive to a common ligand.

6. A method according to claim 4 wherein the DNA binding domain of GAL4 comprises at least the first 74 amino terminal amino acid residues of GAL4.

7. A method according to claim 4 wherein the DNA binding domain of GAL4 is introduced at the amino terminus of the receptor protein.

8. A method according to claim 4 wherein said modified receptor protein consists essentially of amino acid residues 1-147 of GAL4, plus the ligand binding domain of said steroid/thyroid receptor protein.

9. A method of screening for antagonists of steroid/thyroid receptor proteins, said method comprising
culturing test cells containing
(i) increasing concentrations of at least one compound whose ability to inhibit the transcription activation activity of hormone receptor agonists is sought to be determined, and
(ii) optionally, at least one agonist for said steroid/thyroid receptor protein(s) or functional modified forms thereof,
wherein said test cells contain
(i) exogenous DNA which expresses a modified form of a steroid/thyroid receptor protein(s) or functional modified forms thereof, wherein the DNA binding domain of GAL4 replaces the native ligand binding domain of said modified form of the steroid/thyroid receptor protein(s) or functional modified forms thereof, and
(ii) a GAL4 response element operatively linked to a reporter gene; and thereafter
assaying for evidence of transcription of said reporter gene in said cells as a function of the concentration of said compound in said culture medium, thereby indicating the ability of said compound to inhibit activation of transcription by hormone receptor agonists.

10. A method to render a steroid/thyroid receptor protein capable of forming a homodimer, said method comprising replacing the native DNA binding domain of said receptor protein with the DNA binding domain of GAL4.

11. A method according to claim 10 wherein the DNA binding domain of GAL4 comprises at least the first 74 amino terminal amino acid residues of GAL4.

12. A method according to claim 10 wherein the DNA binding domain of GAL4 is introduced at the amino terminus of the receptor protein.

13. A method according to claim 10 wherein said modified receptor protein consists essentially of amino acid residues 1-147 of GAL4, plus the ligand binding domain of said steroid/thyroid receptor protein.

14. A method for identifying biologically active compounds characterized by being:
convertible into ligand or ligand precursor for intracellular receptor(s), or
capable of effecting transformation of a second compound into ligand or ligand precursor for intracellular receptor(s), or
capable of effecting transformation of an intracellular receptor into its active form,
said method comprising:
(1) contacting cells or cell fractions with said biologically active compound under physiological conditions;
(2) fractionating the cells, cell fractions and/or the medium in which said contacting is carried out; and
(3) subjecting each of said fractions to a functional assay wherein each fraction is cultured with host cells containing DNA encoding a chimeric receptor comprising a member of the steroid/thyroid receptor protein superfamily wherein the native DNA binding domain is replaced with the DNA binding domain of the yeast GAL4 protein, and a GAL4 response element operatively linked to reporter-encoded DNA, and expression of reporter gene is monitored to determine the presence of an activated receptor-ligand complex.

15. A method for identifying cell lines which are capable of promoting the conversion of biologically active compounds into:
(A) ligand or ligand precursor for intracellular receptor(s), or
(B) species which promote the transformation of a second compound into ligand or ligand precursor for intracellular receptor(s), or
(C) species which are capable of effecting transformation of an intracellular receptor into its active form,
said method comprising:
(1) contacting cells with biologically active compound(s) under conditions suitable to maintain the viability of said cells;
(2) fractionating the cells and the medium in which the cells are maintained;
(3) subjecting each of said fractions to a functional bioassay wherein each fraction is cultured with host cells containing DNA encoding a chimeric receptor comprising a member of the steroid/thyroid receptor protein superfamily wherein the native DNA binding domain is replaced with the DNA binding domain of yeast GAL4 protein, and a GAL4 response element operatively linked to reporter-encoded DNA, and expression of reporter gene is monitored to determine the presence of an activated receptor-ligand complex; and
(4) identifying those cell lines which promote the conversion of said biologically active compounds into products functional in said functional bioassay.

16. A method for identifying enzymatic or non-enzymatic activities capable of promoting the conversion of biologically active compounds into:
(A) ligand or ligand precursor for intracellular receptor(s), or
(B) species which promote the transformation of a second compound into ligand or ligand precursor for intracellular receptor(s), or
(C) species which are capable of effecting transformation of an intracellular receptor into its active form,
said method comprising:
(1) subjecting biologically active compound(s) to conditions suitable to promote the desired conversion;
(2) subjecting the reaction product(s) of step (1) to a functional bioassay wherein each fraction is cultured with host cells containing DNA encoding a chimeric receptor comprising a member of the steroid/thyroid receptor protein superfamily wherein the native DNA binding domain is replaced with the DNA binding domain of the yeast GAL4 protein, and a GAL4 response element operatively linked to reporter-encoded DNA, and expression of reporter gene is monitored to determine the presence of an activated receptor-ligand complex; and
(3) identifying those conditions which promote the conversion of said biologically active compounds into products functional in said functional assay as providing the desired activities.


SEQUENCE LISTING

(1) GENERAL INFORMATION:

(i) APPLICANT: Evans, Ronald M.
Umesono, Kazuhiko
Blumberg, Bruce
Rangarajan, Pundi N.

(ii) TITLE OF INVENTION: NOVEL USES FOR GAL4-RECEPTOR
CONSTRUCTS

(iii) NUMBER OF SEQUENCES: 5

(iv) CORRESPONDENCE ADDRESS:
(A) ADDRESSEE: Pretty, Schroeder, Brueggemann & Clark
(B) STREET: 444 South Flower Street, Suite 2000
(C) CITY: Los Angeles
(D) STATE: California
(E) COUNTRY: USA
(F) ZIP: 90071

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER: US 08/177,740
(B) FILING DATE: 30-DEC-1993
(C) CLASSIFICATION:

(viii) ATTORNEY/AGENT INFORMATION:
(A) NAME: Reiter, Stephen E.
(B) REGISTRATION NUMBER: 31,192
(C) REFERENCE/DOCKET NUMBER: P41 9392

(ix) TELECOMMUNICATION INFORMATION:
(A) TELEPHONE: 619-546-4737
(B) TELEFAX: 619-546-9392

(2) INFORMATION FOR SEQ ID NO:1:

(i) SEQUENCE CHARACTERISTICS:
(A) LENGTH: 71 amino acids
(B) TYPE: amino acid
(D) TOPOLOGY: linear

(ii) MOLECULE TYPE: protein

(v) FRAGMENT TYPE: N-terminal

(ix) FEATURE:
(A) NAME/KEY: Modified-site
(B) LOCATION: group(37..38, 45..47)
(D) OTHER INFORMATION: /label= Option
/note= "These residues are optional. Thus, the
DNA-binding domain is a minimum of 66 amino acids in
length, but can contain several additional residues."

(ix) FEATURE:
(A) NAME/KEY: Region
(B) LOCATION: one-of(7, 9, 11, 13, 22, 27, 62, 65, 70)
(D) OTHER INFORMATION: /label= Conserved
/note= "These residues are almost universally conserved,
but variations have been found in some identified hormone
receptors."

(xi) SEQUENCE DESCRIPTION: SEQ ID NO:1:

Cys Xaa Xaa Cys Xaa Xaa Asn Xaa Ala Xaa Gly Xaa Tyr Xaa Xaa Xaa
1               5                   10                  15

Xaa Cys Xaa Xaa Cys Lys Xaa Phe Phe Xaa Arg Xaa Xaa Xaa Xaa Xaa
            20                  25                  30

Xaa Xaa Xaa Xaa Xaa Xaa Cys Xaa Xaa Xaa Xaa Xaa Xaa Xaa Xaa Cys
        35                  40                  45

Xaa Xaa Xaa Lys Xaa Xaa Arg Xaa Xaa Cys Xaa Xaa Cys Arg Xaa Xaa
    50                  55                  60

Lys Cys Xaa Xaa Xaa Gly Met
65                  70

(2) INFORMATION FOR SEQ ID NO:2:

(i) SEQUENCE CHARACTERISTICS:
(A) LENGTH: 546 base pairs
(B) TYPE: nucleic acid
(C) STRANDEDNESS: double
(D) TOPOLOGY: both

(ii) MOLECULE TYPE: DNA (genomic)

(iii) HYPOTHETICAL: NO

(iv) ANTI-SENSE: NO

(ix) FEATURE:
(A) NAME/KEY: CDS
(B) LOCATION: join(35..532, 536..544)

(xi) SEQUENCE DESCRIPTION: SEQ ID NO:2:

GGGAGACCCA AGCTTGAAGC AAGCCTCCTG AAAG ATG AAG CTA CTG TCT TCT      52
                                      Met Lys Leu Leu Ser Ser
                                        1               5

ATC GAA CAA GCA TGC GAT ATT TGC CGA CTT AAA AAG CTC AAG TGC TCC     100
Ile Glu Gln Ala Cys Asp Ile Cys Arg Leu Lys Lys Leu Lys Cys Ser
            10                  15                  20

AAA GAA AAA CCG AAG TGC GCC AAG TGT CTG AAG AAC AAC TGG GAG TGT     148
Lys Glu Lys Pro Lys Cys Ala Lys Cys Leu Lys Asn Asn Trp Glu Cys
        25                  30                  35

CGC TAC TCT CCC AAA ACC AAA AGG TCT CCG CTG ACT AGG GCA CAT CTG     196
Arg Tyr Ser Pro Lys Thr Lys Arg Ser Pro Leu Thr Arg Ala His Leu
    40                  45                  50

ACA GAA GTG GAA TCA AGG CTA GAA AGA CTG GAA CAG CTA TTT CTA CTG     244
Thr Glu Val Glu Ser Arg Leu Glu Arg Leu Glu Gln Leu Phe Leu Leu
55                  60                  65                  70

ATT TTT CCT CGA GAA GAC CTT GAC ATG ATT TTG AAA ATG GAT TCT TTA     292
Ile Phe Pro Arg Glu Asp Leu Asp Met Ile Leu Lys Met Asp Ser Leu
                75                  80                  85

CAG GAT ATA AAA GCA TTG TTA ACA GGA TTA TTT GTA CAA GAT AAT GTG     340
Gln Asp Ile Lys Ala Leu Leu Thr Gly Leu Phe Val Gln Asp Asn Val
            90                  95                  100

AAT AAA GAT GCC GTC ACA GAT AGA TTG GCT TCA GTG GAG ACT GAT ATG     388
Asn Lys Asp Ala Val Thr Asp Arg Leu Ala Ser Val Glu Thr Asp Met
        105                 110                 115

CCT CTA ACA TTG AGA CAG CAT AGA ATA AGT GCG ACA TCA TCA TCG GAA     436
Pro Leu Thr Leu Arg Gln His Arg Ile Ser Ala Thr Ser Ser Ser Glu
    120                 125                 130

GAG AGT AGT AAC AAA GGT CAA AGA CAG TTG ACT GTA TCG CCG GAA TTC     484
Glu Ser Ser Asn Lys Gly Gln Arg Gln Leu Thr Val Ser Pro Glu Phe
135                 140                 145                 150

CCG GGG ATC CGT CGA CGG TAC CAG ATA TCA GGA TCC TGG CCA GCT AGC     532
Pro Gly Ile Arg Arg Arg Tyr Gln Ile Ser Gly Ser Trp Pro Ala Ser
                155                 160                 165

TAG GTA GCT AGA GG                                                  546
    Val Ala Arg

(2) INFORMATION FOR SEQ ID NO:3:

     (i) SEQUENCE CHARACTERISTICS:
          (A) LENGTH: 169 amino acids
          (B) TYPE: amino acid
          (D) TOPOLOGY: linear

    (ii) MOLECULE TYPE: protein

    (xi) SEQUENCE DESCRIPTION: SEQ ID NO:3:

Met Lys Leu Leu Ser Ser Ile Glu Gln Ala Cys Asp Ile Cys Arg Leu
  1               5                  10                  15

Lys Lys Leu Lys Cys Ser Lys Glu Lys Pro Lys Cys Ala Lys Cys Leu
             20                  25                  30

Lys Asn Asn Trp Glu Cys Arg Tyr Ser Pro Lys Thr Lys Arg Ser Pro
         35                  40                  45

Leu Thr Arg Ala His Leu Thr Glu Val Glu Ser Arg Leu Glu Arg Leu
     50                  55                  60

Glu Gln Leu Phe Leu Leu Ile Phe Pro Arg Glu Asp Leu Asp Met Ile
 65                  70                  75                  80

Leu Lys Met Asp Ser Leu Gln Asp Ile Lys Ala Leu Leu Thr Gly Leu
                 85                  90                  95

Phe Val Gln Asp Asn Val Asn Lys Asp Ala Val Thr Asp Arg Leu Ala
            100                 105                 110

Ser Val Glu Thr Asp Met Pro Leu Thr Leu Arg Gln His Arg Ile Ser
        115                 120                 125

Ala Thr Ser Ser Ser Glu Glu Ser Ser Asn Lys Gly Gln Arg Gln Leu
    130                 135                 140

Thr Val Ser Pro Glu Phe Pro Gly Ile Arg Arg Arg Tyr Gln Ile Ser
145                 150                 155                 160

Gly Ser Trp Pro Ala Ser Val Ala Arg
                165

(2) INFORMATION FOR SEQ ID NO:4:

(i) SEQUENCE CHARACTERISTICS:
(A) LENGTH: 57 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:4:

CCGGAATTCG GTACCGTCGA CGATATCCCC CGGGCTGCAG GATCCTGGCC AGCTAGC 57

(2) INFORMATION FOR SEQ ID NO:5:

(i) SEQUENCE CHARACTERISTICS:
(A) LENGTH: 17 base pairs
(B) TYPE: nucleic acid
(C) STRANDEDNESS: both
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:5:

CGGAGGACTG TCCTCCG 17